# EUROPEAN PATENT APPLICATION

(11) **EP 0 619 115 A1**
(43) Date of publication of application: **12.10.1994**
(21) Application number: 94105074.2
(22) Date of filing: 31.03.1994
(51) Int. Cl.: A61K 9/12, A61K 9/16

(54) **Container comprising a metered dose valve and microparticles of a drug for topical treatment of skin disorders**

(30) Priority: 01.04.1993 US 41075
(71) Applicant: AMGEN INC., Thousand Oaks, CA 91320-1789 (US)
(72) Inventor: Cha, Younsik, Thousand Oaks, California 91360 (US)
(74) Representative: Vossius, Volker, Dr.

(57) **Abstract**

Disclosed is a container for delivering a preset amount of a drug for topical treatment of skin disorders.

## Description

This invention relates to methods of administration of drugs. More specifically, the invention concerns topical administration of preset doses of drugs useful for treating skin disorders and for wound healing.

A preferred method of applying drugs used to treat skin disorders and wounds is topical application. This method avoids possible side effects often associated with drugs administered orally or by injection. Oral administration and injection result in exposure of many bodily tissues to the drug, while topical or local administration exposes essentially only the tissues to which the drug is applied.

Methods available for topical or local application of drugs include the use of creams, gels, liquid solutions, and sprays, or the implantation of strips containing medication (in the case of surgical wounds/incisions). While these methods are adequate in many cases, they are each limited in their usefulness.

Application of a gel or cream to the affected area of skin requires touching the skin, which may further irritate the skin and/or introduce dirt or pathogens to the skin. In addition, delivery of a uniform and precise dose of the drug can be difficult.

A liquid formulation may run off of the targeted area of skin unless it is absorbed quickly and/or the skin is held in a proper position until the liquid is fully absorbed. Thus, delivery of a precise dose of the drug to the target area can be difficult using this method.

Implantation of medicated strips into surgical incisions is not a favored method of application of a drug for wound healing, as this method involves placing a foreign object into the wound. The object may become displaced, or may cause an infection.

Application of a spray solution to the affected area of skin prevents the need to touch the skin. However, unless the spray dries rapidly or is absorbed quickly by the skin, the solution may run off the targeted area of skin, thereby decreasing the dose of the drug actually delivered to the skin.

Platelet derived growth factor (PDGF) is a protein produced naturally in many mammals including humans. Among other biological activities, PDGF appears to stimulate healing and tissue repair of various types of dermal and surgical wounds when administered at appropriate concentrations, usually about 0.5-5.0 µg/cm² (Grotendorst *et al., J. Clin. Invest.*, 76:2323-2329 [1985]; *Robson et al., The Lancet*, 339:23-25 [1992]; Pierce, G.F. in *Encyclopedia of Human Biology*, vol. 7, pp. 499-509, Academic Press [1991]).

In its naturally occurring form, PDGF exists as a disulfide-bonded dimer of two polypeptide chains, termed the A chain and the B chain. The amino acid sequences of the A and B chains share about sixty percent homology, and both contain eight cysteine residues. Three distinct dimeric forms of PDGF exist, the PDGF-AB heterodimer, the PDGF-AA homodimer, and the PDGF-BB homodimer (see Hannick *et al., Mol. Cell. Biol.*, 6:1304-1314 [1986]). Although PDGF-AB has been identified as the predominate naturally occurring form, PDGF-BB has been most widely used in wound healing studies.

The PDGF-B chain found in human platelets has been identified as a 109 amino acid cleavage product of a 241 amino acid precursor polypeptide (Johnsson *et al., EMBO Journal*, 3:921-928 [1984]). This 109 amino acid polypeptide (PDGF-B₁₀₉) is homologous with amino acids 82-191 of *c-sis*, the gene encoding the PDGF-B precursor polypeptide. The full length of *c-sis* is 241 amino acids. Another form of PDGF-B (PDGF-B₁₁₉) corresponding with amino acids 82-201 of the *c-sis* gene product has also been identified as a major cleavage product of the c-sis encoded precursor product when the entire *c-sis* gene is transfected into, and expressed in, a mammalian host cell.

European Patent No. 243,179, published October 28, 1992, describes a composition comprised of collagen, heparin or a glycosaminoglycan, in combination with either a chemotactic factor, a growth factor such as epidermal growth factor or platelet derived growth factor, or a differentiation factor. The composition is reportedly useful for healing soft tissue wounds.

U.S. Patent No. 4,973,466, issued November 27, 1990, describes a wound healing dressing made of fibronectin that has been flocculated to produce a water swellable gel which is applied to the wound.

U.S. Patent No. 4,760,131, issued July 26, 1988, describes a composition that reportedly is useful for soft tissue wound healing. The composition is comprised of collagen, heparin or a glycosaminoglycan, and undegranulated platelets or platelet releasate.

U.K. patent application, GB 2,245,831, published January 15, 1992, describes loading beta-fibroblast growth factor into water-insoluble microspheres made of cellulose, gelatin, collagen, dextran or starch derivatives. The complex is lyophilized to form a powder, and the powder is applied directly to wound or burn sites on the skin.

European Patent No. 440,989, published December 29, 1990, teaches a method for preparing a dried composition of insulin-like growth factor I by drying it in the presence of a strong acid.

European Patent No. 412,554, published February 13, 1991, describes sustained release formulations of drugs such as neurotrophic factors, cell growth factors such as epidermal growth factor and platelet derived growth factor, neurotransmitters, and related compounds useful in treating cerebral disorders by incorporating the drug into a biodegradable carrier such as glycolic or lactic acid, albumin, collagen, or gelatin.

U.S. Patent No. 4,962,091, issued October 9, 1990, describes a delivery system for controlled administration of a drug. The system includes a polylactide matrix into which polypeptides are dispersed.

U.S. Patent No. 4,743,583, issued May 10, 1988, discloses a sustained release delivery system for polypeptides. The polypeptides are dispersed in a solution containing a non-aqueous Lewis base and an aqueous Lewis acid to produce an emulsion of microdroplets which are then collected by centrifugation.

U.S. Patent No. 4,659,570, issued April 21, 1987, discloses a stabilized preparation of a polypeptide admixed with chemically modified gelatin.

U.S. Patent No. 5,011,678, issued April 30, 1991, teaches compositions for transmucosal administration of drugs, comprised of a polypeptide-type or other drug, and a biocompatible steroid, in combination with an aerosol propellant.

Canadian Patent Application 2,025,282, published March 14, 1992, describes a method for preparing an aerosol formulation of collagen, optionally containing a carrier such as gelatin and a pharmacologically active agent such as platelet derived growth factor. The formulation is reportedly useful as a wound dressing.

WO 91/16882, published November 14, 1991, discloses a method of preparing a drug-lipid powder composition for water soluble drugs. The powder composition can be administered to the respiratory tract at selected doses by producing an airborne suspension.

WO 91/14422, published October 3, 1991, describes aerosol formulations comprised of a hydrocarbon propellant, a powdered drug, and a dispersing agent. The powdered drug can be a hormone, enzyme, peptide, steroid, antibiotic or other compound, and is prepared in a micronized form such that most of the particles have a diameter of less than about 10 microns. The aerosol formulations are reportedly suitable for dermal, pulmonary or mucosal administration.

U.S. Patent No. 4,892,889, issued January 9, 1990, describes a process for preparing a vitamin powder by spray drying a solution of fat soluble vitamins, gelatin, and water soluble carbohydrates.

U.S. Patent No. 4,734,401, issued March 29, 1988, describes a process for preparing a dried composition of one or more amino acids by spray drying.

U.S. Patent No. 4,233,405, issued November 11, 1980, describes a process for preparing spray dried enzyme compositions. The process involves concentrating a liquid composition of enzyme and water insoluble salts, and spray drying this composition at elevated temperatures.

U.S. Patent No. 3,207,666, issued September 21, 1965, describes a method for forming a dry, free flowing powder containing a highly oxidizable organic substance. A solution of the substance is atomized in the presence of an organic film-forming colloid, an antioxidant, and a carbohydrate to form droplets that serve to protect the oxidizable organic substance from oxidation.

The use of spray applicators for administration of certain drugs that are topically applied is known in the art. For example, Polysporin® brand antibiotic ointment (Burroughs Wellcome, Inc., Research Triangle Park, N.C.) and Decaspray® brand steroid spray (Merck, Sharp & Dohme, Inc., West Point, PA) are both packaged in spray applicators. The problem with these and other spray-type containers is that it is not possible to administer an accurate dose of drug to the targeted skin area; dosage is usually measured by holding the can a certain distance from the skin and spraying the drug for a specified amount of time.

There is a need in the art for spray applicators that deliver to targeted areas accurate and precise doses of drugs used to treat skin disorders such as wounds and surgical incisions.

Accordingly, an object of the invention is to provide a method for dispensing a drug used to treat skin disorders such as wounds and surgical incisions using a spray applicator that delivers a preset dose of the drug to the targeted area of skin.

A further object is to provide suitable formulations of the drug for packaging and dispensing in an aerosol sprayer.

These and other objects will readily be apparent to one of ordinary skill in the art.

### SUMMARY OF THE INVENTION

This invention is based on the discovery that formulations of drugs used for treating skin disorders and for wound healing can be prepared and administered in preset doses to deliver an accurate amount of drug to the skin using a spray-type applicator to which is attached a metered dose valve. The invention thus avoids problems encountered with the use of gels, creams, or liquid formulations used for topical application.

In one preferred embodiment, this invention provides a container comprising a metered dose valve and microparticles of a drug used to treat a skin disorder.

In another embodiment, the invention provides microparticles of a drug that have PDGF-like activity.

In another preferred embodiment, the drug with PDGF -like activity is human recombinant PDGF-BB formed into microparticles with an excipient, and the microparticles are packaged in the container with a propellant and optionally a lubricant.

In yet another preferred embodiment, the excipient is selected from hydrolyzed gelatin with an average molecular weight of 20,000 daltons, collagen, and carbohydrate.

In still another preferred embodiment, the lubricant is isopropyl myristate, and the propellant is isobutane.

In one other preferred embodiment, the invention provides a method of preparing a device to deliver a preset dose of a drug comprising preparing microparticles comprising a drug used to treat a skin disorder, packaging the microparticles in a container, sealing the container with a metered dose valve, and adding propellant to the container.

In still one other preferred embodiment, the invention provides a method of promoting wound or surgical incision healing comprising administering a drug with PDGF-like activity from a container comprising a metered dose valve.

### BRIEF DESCRIPTION OF THE DRAWINGS

Figure 1 is a schematic diagram of a suitable spray drier apparatus used to prepare microparticles.

Figure 2 depicts a container with an inverted metered dose valve. As depicted, the container fits into an actuator. The actuator has finger rests and a long nozzle tip.

### DETAILED DESCRIPTION OF THE INVENTION

The following terms are used to describe the invention.

The terms "excipient" and "excipients" refer to any chemical compound that is pharmaceutically inactive or inert, and serves as a bulking agent to prevent undesirable hygroscopic or other physical interactions between molecules of the drug. The excipient may also be useful as a dilution agent and for ease of handling the drug. The excipient may be a naturally occurring compound, or may be one that is manufactured synthetically by any means. Preferred excipients will have particular average molecular weights; this is important for obtaining microparticles of the appropriate size. For protein based excipients, a molecular weight of about 10,000 daltons to about 30,000 daltons is preferred for most applications.

For certain applications, it may be desirable to use more than one excipient simultaneously. Examples of suitable excipients include, without limitation, soluble collagen (obtained from any source), gelatin, and carbohydrates. Preferred excipients are collagen and gelatin. The most preferred excipient is gelatin.

The terms "microparticle" and "microparticles" refer to the form that the drug-excipient mixture or matrix takes on when an aqueous solution of the drug-excipient mixture is passed through a spray drier apparatus. As the solution passes through the nozzle of the spray drier, a fine mist is formed, and the water in the mist evaporates resulting in production of dried microparticles of the drug-excipient complex. Generally, microparticles are about 150 microns or less in diameter, preferably about 50 microns or less in diameter, and most preferably about 5-15 microns in diameter.

The term "PDGF" refers to platelet derived growth factor or any peptide or polypeptide exhibiting PDGF like activity. As used herein, the PDGF can be from any source*, i.e.*, human or non-human mammal, or manufactured by a synthetic or semi-synthetic means, such as for example, by recombinant DNA technology or by peptide synthesis. Included within the scope of the invention are insertion, deletion and/or substitution mutants of PDGF. Both the heterodimeric PDGF-AB form and the homodimeric forms PDGF-BB and PDGF-AA are within the scope of this invention. Both the 109 and 119 amino acid forms of PDGF-B are within the scope of this invention, as are biologically active fragments thereof. Human recombinant PDGF-BB of length 119 amino acids is a preferred form of PDGF for use in practicing this invention. As used herein, the term is meant to include any biologically active fragments of PDGF-AB, PDGF-BB, PDGF-AA, and/or fragments of the A or B monomers. Where PDGF is used for topical application to promote wound healing, the dosage administered may vary depending on the form of the PDGF used. For the mature form of the PDGF-BB protein, a dosage of between about 0.5 and 5 µg per cm² of wound is suitable, however the dosage is preferably about 1.5 to 3.5 µg per cm² of wound, and most preferably about 2-2.5 µg per cm² of wound. Suitable dosages for other forms of PDGF can be determined using routine experimentation by methods well known to those of skill in the art.

The terms "skin disorder" and "dermal disorder" are meant to include any type of disease of the skin, or trauma to any part of the skin, either directly or indirectly. Examples of such disorders include, without limitation, skin cancers, dermatitis, rashes, allergic reactions, acne, wounds, dermal ulcers (pressure ulcers, venous stasis ulcers, and diabetic ulcers, for example) surgical incisions, and the like.

### Methods of Making the Invention

### 1. Source of the Drug

Any drug used to practice this invention should be soluble in an aqueous medium, and fairly stable at elevated temperatures, *i.e.*, retain at least partial biological activity at temperatures between about 100°C and 150°C. The drug may be organic or inorganic, and may be derived from any natural source, or manufactured synthetically. Drugs may be protein, carbohydrate, lipid, nucleic acid, or may be any other type of organic chemical molecule such as, for example, a heterocyclic, aromatic, or hydrocarbon compound.

This invention contemplates the use of more than one drug in a given microparticle formulation, provided that the drugs, when combined in a single formulation, are chemically and pharmaceutically compatible.

Drugs that are proteins can be obtained by purification of the protein from any endogenous source such as a particular cell-type or tissue of vertebrate animals, invertebrate animals, or plants. Methods for such purification are well known to the skilled artisan, such as those described in Sambrook *et al*. (*Molecular Cloning: A Laboratory Manual*, Cold Spring Harbor Laboratory Press, Cold Spring Harbor, NY. [1989]). Alternatively or additionally, the protein may be obtained using recombinant DNA technology procedures that are generally well known to the skilled artisan. Where recombinant technology is used to obtain a protein, this invention contemplates the use of biologically active fragments of the protein, as well as mutants of the protein such as insertion, deletion, and/or substitution mutants.

Non-protein drugs can be obtained through any means known in the art, such as by purification from a suitable source, or by chemical or enzymatic synthesis.

Preferred drugs are those that promote wound healing or are useful in treating wounds and surgical incisions, as well as skin disorders such as various skin cancers, dermatitis, rashes, allergic reactions, and various types of acne. Drugs such as steroids, antibiotics, growth factors, including without limitation keratinocyte growth factor (KGF) and platelet derived growth factor (PDGF), are more preferred. The most preferred drug is PDGF-BB in the 119 amino acid sequence form.

### 2. Source of the Excipient

The excipient can be any pharmaceutically inactive substance, organic or inorganic, that is useful both as a dilution agent and to decrease or prevent undesirable interactions between particles of the drug, such as aggregation, hygroscopic interactions, and the like. Useful excipients are well known in the art (see, for example, *Remington's Pharmaceutical Sciences*, 17th ed., 1985, Mack publishers, Easton, PA). The excipients may be in the naturally occurring form, or may be chemically, enzymatically or otherwise modified. Generally, excipients with a smaller molecular weight (less than about 25,000 daltons for proteins) are preferred, as smaller microparticles can more readily be prepared from them.

More than one excipient may be used simultaneously in a given formulation. The amount of excipient used per unit of drug will depend on the characteristics of the drug, the desired concentration of the drug, and the dose of the drug to be delivered to the target area of skin.

Preferred excipients for use in the invention are various carbohydrates such as lactose, glucose, mannitol or hydroxyethyl starch, or proteins such as human serum albumin, collagen, or gelatin. More preferred excipients are collagen and gelatin, and the most preferred excipient is gelatin hydrolyzed to an average molecular weight of about 20,000 daltons.

### 3. Sustained Release Formulations

In some cases, it may be advantageous to apply a sustained release formulation of the drug to the target tissue. This may serve to decrease the frequency of applications of the drug. In these cases, certain polymers such as human serum albumin, cellulose, starch or derivatives thereof, or synthetic polymers can be used as a matrix for a sustained release formulation. These polymers must be pharmacologically inert and non-toxic. The desired amount of polymer is added to the formulation. The amount of polymer used will be determined empirically, but will primarily be dependent on its chemical composition, its rate of degradation when applied to skin, the amount of drug present in the formulation, and other similar considerations known to those of skill in the art.

It is known that a drug formulation for wound healing containing the protein epidermal growth factor (EGF) in combination with gelatin is more efficient for promoting wound healing than EGF applied alone. The gelatin is believed to decrease the rate at which the EGF is degraded by proteases present at the wound site(Okumura *et al.*, Pharm. Res. 7:1289-1293 [1990]). By analogy, but without being limited to any one theory, it is believed that PDGF would likely be more efficacious or longer lasting when formulated with gelatin or a comparable matrix. Thus, in the present invention, gelatin would be useful both as an excipient, and to extend the life of PDGF applied to the skin for healing wounds or incisions, or for other purposes as previously discussed.

### 4. Preparation of Protein-Excipient Mixture

Typically, the drug-excipient mixture will be prepared using sterile techniques and conditions well known to those of skill in the art. Generally, the drug or drugs to be prepared as microparticles are dissolved in an aqueous medium, preferably purified water, although in certain cases, salts, buffers, and/or other ingredients such as preservatives may also be included as is desired. The concentration of the drug(s) in the aqueous medium is primarily determined based on the final desired microparticle concentration or ratio of drug to excipient, which in turn is calculated based on the desired amount of drug to be delivered per unit amount of spray that will be applied to the skin.

The excipient is dissolved in an aqueous medium containing other ingredients as appropriate. The amount of excipient used is determined in part by the nature of the drug, the amount of dilution of the drug that is desired, and the type of excipient used. The drug and excipient solutions are then mixed together, and kept at or below room temperature.

Alternatively, the drug(s) and excipient may be combined initially into one aqueous solution as a single, complete mixture. This mixture is then kept at or below room temperature.

In addition to excipient, other ingredients such as salts, buffers, preservatives, antibiotics, and the like may be added to the formulation, provided that they are pharmaceutically compatible with the drugs and excipient, and provided they are essentially non-toxic.

### 5. Formation of Microparticles

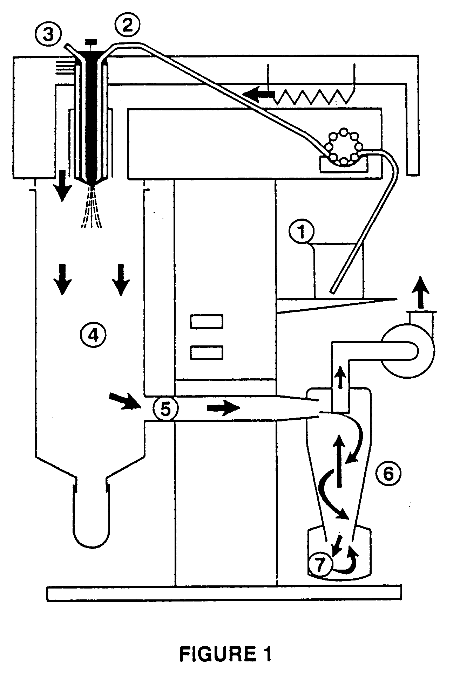

The solution of excipient and drug(s) is spray dried, typically under aseptic conditions, to form microparticles. Spray drying is accomplished using any type of commercially available spray drier apparatus that has a nozzle that sprays or atomizes the solution into a very fine mist. A schematic diagram of such an apparatus is shown in Figure 1, and the following description is best understood by referring to this Figure. The solution of drug and excipient (1) is drawn in to the drying chamber (4) through the nozzle (2). Heated air passes into the drying chamber from inlet (3). The microparticles and hot air are drawn into the cyclone (6) through the aspirator (5). The air passes out through the top of the cyclone, while the dried microparticles are drawn to the bottom of the cyclone and collected into a container (7).

Preferably, the nozzle is kept below room temperature through the use of a water jacket or other appropriate device. The nozzle is enclosed in a drying chamber to which an inlet and an outlet are attached. Hot air at a temperature between about 100°C and 150°C is passed through the inlet into the chamber and serves to heat the atomized particles released from the nozzle, thereby evaporating the water from them to produce solid microparticles.

An aspirator connected to the outlet draws both the heated air and the solid microparticles out of the drying chamber and into a second chamber where the solid microparticles are collected into a small receptacle.

The size of the microparticles can be determined using standard scanning electron microscopy methods.

### 6. Preparation of Aerosol Formulation

A desired amount of the microparticle solid powder is placed in a container for packaging. To the powder may optionally be added a lubricant such as, for example, isopropyl myristate or an equivalent thereof, at a ratio of about 100 mg powder to about 30 µl lubricant (although this ratio may vary where another lubricant is used, or otherwise as desired). Other lubricants that may be used but are less desirable include for example, oleic acid and sorbitan trioleate. The lubricant used is preferably pharmaceutically inactive and fairly non-toxic to the skin.

The container is crimped with a suitable valve using standard machinery such as the crimper and propellant filler machine supplied by Pamasol (BLM Associates Inc., Greenwich, CT; model P2005). The propellant to be added to the container is compressed with a pump (such as the Pamasol model P2008; BLM Associates Inc., Greenwich, CT) and a specified volume of this propellant is dispersed into the container by the crimper and propellant filler machine, following the manufacturer's guidelines in using the machine..

An alternate means of filling the container is the "Cold Filling" method. The microparticles are mixed with a small amount of the propellant to make a concentrate. The concentrate is then cooled down to a temperature that is less than the boiling point of the propellant. This concentrate is then placed in the container, the container is crimped with the valve, and the remainder of the propellant is then added.

The propellant used to practice this invention can be of any type that is used in the pharmaceutical industry. However, most propellants containing fluorocarbons are not preferred due to their detrimental effect on the earth's ozone layer. One fluorocarbon propellant that may be used herein and is not believed to detrimentally affect the ozone layer is 1,1,1-trifluoro-2-fluoro-ethane.

Preferred propellants for use herein are those not containing fluorocarbons that have a vapor pressure between about 15 and 50 psig. One preferred propellant is isobutane. When propellants with vapor pressures outside of this range are used, butane can be added to decrease the vapor pressure, and propane can be added to increase the vapor pressure as is necessary. The amount of propellant used is primarily a function of the desired concentration of drug in the propellant. This in turn is primarily a function of the dose of drug to be applied to the skin per unit of spray administered. Generally, the ratio of drug to propellant will be between about 5-75 mg/ml for PDGF microparticles, and preferably it is about 8-60 mg/ml for PDGF microparticles.

### 7. Dosage Delivery

A key feature of the present invention resides in the use of a metered dose valve with a fixed volume chamber for precise and accurate delivery of a preset amount of the drug to the targeted skin area. The use of this type of valve is important where the amount of drug applied is critical, such as for wound or surgical incision healing.

Any commercially available metered dose valve may be used to practice the invention, provided that it has a rubber gasket or other type of gasket that is chemically inert with respect to the propellant, the lubricant, and the drug-excipient microparticles. The valve may be of the upright or the inverted type, although the inverted type is preferred for ease of administration. Both preset volume and adjustable volume metered dose valves may be used. However, preset volume valves are preferred, especially the inverted metered dose preset volume valves. The preferred valve for use herein is one that delivers about 100 µl per spray, such as the Valois 20 mm inverted valve (Valois of America Inc., obtained from BLM Associates Inc., Greenwich, CT; type DF 10/100 RC 20mm). Other suitable valves are those manufactured by Bespak, Inc. (Framingham, MA).

Containers used for packaging the microparticle-propellant solution may be made of any material that is inert with respect to the propellant and the drug-excipient microparticles. The container must have an opening of a size that is compatible with the size of the metered dose valve, generally about a 20mm circumference, for convenience of packaging. Preferred containers are those made of aluminum, preferably those having a suitable polymer coating. A preferred container is the Safet Canister 20.9 x 45 x 20 mm with an inside lining made of ONC epoxy phenolic (BLM Associates, Greenwich, CT).

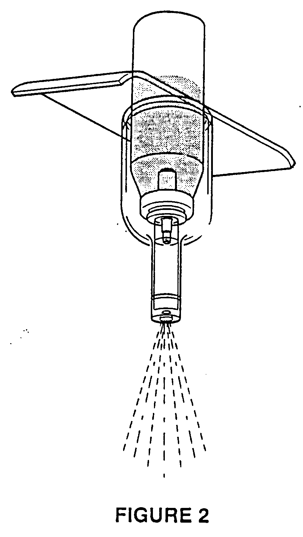

An actuator is affixed to the container-metered dose valve apparatus. The actuator may be any type that is compatible with the metered dose valve selected for use. A preferred actuator is one that has finger rests and a long nozzle. The finger rests provide a means of holding the apparatus comfortably when the apparatus is used in an inverted position for administration, and the long nozzle aids in delivering the drug in a precise manner. This apparatus comprising the container, inverted metered dose valve and actuator is diagrammed in Figure 2.

### 8. Stability of Formulation

The stability of the formulation over time is important for maintaining the efficacy of the drug. Stability can be monitored using *in vivo* or *in vitro* assays designed to evaluate the activity of the drug. The assays may be conducted by storing a container of the prepared formulation under appropriate temperature conditions for a period of time, and evaluating the biological activity of the formulation at several points during that time.

The invention will be more fully understood by reference to the following examples. These examples should not be construed in any way as limiting the scope of this invention.

### EXAMPLE I

### 1. Preparation of PDGF Microparticles

### a. PDGF-Gelatin Microparticles

An aqueous solution of human recombinant PDGF-BB was dialyzed against 10 mM Na acetate, pH 4.0, to remove salts from the solution. The dialyzed solution (50 ml) at a concentration of 2.76 mg/ml PDGF was mixed with a previously filtered solution of 13.66 g of hydrolyzed purified gelatin with an average molecular weight of 20,000 daltons (obtained from Dynagel, Inc., Calumet City, Illinois) in 86.6 ml of water, to yield a final solution of 10 percent by weight of gelatin and 1 percent by weight of PDGF.

### b. PDGF-Collagen Microparticles

10 ml of an aqueous solution of 2.038 mg/ml human recombinant PDGF-BB in 10 mM Na acetate, 150 mM NaCl, pH 4.0 was added to 100 ml of a solution of Semed S soluble collagen (Semex Medical Co., Malvern, PA). The collagen was previously prepared at a concentration of 1.0 g/100 ml of 0.1 M acetic acid.

### c. Making the Microparticles

The solutions of PDGF-hydrolyzed gelatin and PDGF-collagen were spray dried using a Buchi mini spray drier (Brinkman Instruments, model 190). The spray drier was modified by placing a circulating water jacket around the nozzle to keep the solution entering the nozzle at about 20°C. The air that was passed into the drying chamber via the inlet was first filtered for purposes of maintaining aseptic conditions. The air inlet temperature was about 136°C, the outlet temperature was about 96°C, the aspirator was set at maximum, and the air spray was set at 600 liters/hour. Solid microparticles were formed as the gelatin-PDGF or collagen-PDGF solution was passed through the nozzle and the water was evaporated from the misted particles. The microparticles were drawn into the cyclone portion of the spray drier (see Figure 1), and collected into a sterile receptacle.

Scanning electron microscopy was used to evaluate the size of the microparticles generated by this method. On average, the microparticles ranged from 1-10 microns in diameter.

The microparticles were stored in a dessicator at room temperature.

### 2. Preparation of Aerosol Formulation

Five hundred milligrams of microparticles and 150 µl of isopropyl myristate were placed in a Wheaton plastic coated glass bottle, and the bottle was sealed with an inverted metered dose valve (Valois, DF 10/100, #400; BLM Associates, Inc., Greenwich, CT) using the Pamasol P2005 crimping and pressure filling equipment (BLM Associates, Inc.) and following the manufacturer's instructions. Ten milliliters of isobutane, pressurized using the Pamasol P2008 propellant compressor pump, was added to the bottle through the valve, again using the Pamasol P2005 pressure and filling equipment. An actuator (Valois 25 Gpp 0/7) was installed, and the packaged solution was stored at room temperature.

### 3. PDGF Microparticle Stability Assays

The stability of PDGF-BB-hydrolyzed gelatin microparticles over a two week period in both the powder and aerosol forms was evaluated by non-denaturing SDS polyacrylamide gel electrophoresis (SDS-PAGE) and Western blot analysis, using standard techniques well known to the skilled artisan.

Two gels were run simultaneously; one was Coomassie stained for protein, and the other was Western blotted. Each gel was a 10-20 percent gradient gel (10 cm x 10 cm; obtained from Integrated Separation Systems, Enprotech, Inc., Hyde Park, MA) and was run according to manufacturer's instructions at about 35 mA constant current. The aerosol formulation samples were prepared as follows: Glass plates were sprayed with the aerosol formulation, and about 26 mg of powder were collected from the plates. The powder was dissolved in about 1 ml of 10 mM Na acetate buffer, pH 4.0; this resulted in a PDGF concentration of about 0.2 mg/ml. This sample was then diluted with the same buffer for a final PDGF concentration Of about 0.1 mg/ml. Six microliters of this sample were mixed with 2 µl of electrophoresis buffer containing SDS and dye, and this sample was loaded on to the gel.

The microparticle powder samples were prepared by adding about 50 mg dry powder to about 2.5 ml of buffer (10 mM Na acetate, pH 4.0). This solution was diluted two fold with the same buffer to obtain a final concentration of about 0.1 mg/ml PDGF. About 6 µl of this sample were added to 2 µl of buffer containing SDS and dye, and this sample was then loaded on to the gels. After the gels were run, one gel was stained with Coomassie blue dye using standard procedures, and the other was Western blotted on to nitrocellulose paper using the following procedure.

The gel to be blotted was soaked for 20 minutes at room temperature in Kodak EZE Formula I buffer (Eastman Kodak Company, Rochester, NY). The pH of the buffer was adjusted to about 9.5 by adding 10 N NaOH. Prior to the transfer, the nitrocellulose paper was soaked in methanol for about 5 minutes, and then in cold (about 4°C) buffer (Kodak EZE Formula I) for about 15 minutes. The transfer was conducted for about one hour at 125 mA using the ISS Enprotech semi-dry blotting unit and following manufacturer's instructions. The Western blot was then dried and probed with antibody.

The probe used for PDGF protein detection on the Western blot was a mouse monoclonal antibody diluted into a solution of PBS (phosphate buffered saline, obtained as a 10x stock from Irvine Scientific, Santa Ana, CA, and prepared according to manufacturer's instructions) and 10 percent horse serum at a ratio of about 1:250. This incubation was done overnight at room temperature on a shaker. After the incubation, the blot was washed in PBS. The assay to detect the presence of the mouse monoclonal antibody was conducted with a Vectastain kit (Vector Laboratories, Burlingame, CA) following the manufacturer's instructions. A biotinylated anti-mouse antibody was added to the blot and incubated for about one hour at room temperature; the blot was then washed in PBS. A solution containing biotinylated horse radish peroxidase and avidin was then added. The horse radish peroxidase was detected by adding color developer.

No noticeable degradation of PDGF was observed either from the Comassie stained gel, or from the Western blot, indicating that the microparticles remained stable over at least a two week period, whether they were stored dry, or in a spray container in the presence of propellant.

## Claims

1. A container comprising a metered dose valve and microparticles of a drug for topical treatment of skin disorders.

2. The container of claim 1 wherein the drug possesses human PDGF-like activity.

3. The container of claim 2 wherein the drug is human recombinant PDGF-BB.

4. The container of claim 2 or 3 further comprising an excipient selected from the group consisting of: collagen, gelatin, and carbohydrate.

5. The container of claim 4 wherein the excipient is hydrolyzed gelatin with an average molecular weight of about 20,000 daltons.

6. The container of claim 5 further comprising a propellant and a lubricant.

7. The container of claim 5 that comprises isobutane, isopropyl myristate, and human recombinant PDGF-BB-hydrolyzed gelatin microparticles, wherein the hydrolyzed gelatin has an average molecular weight of about 20,000 daltons.

8. A method of preparing a device to deliver a preset dose of a drug comprising:
(a) preparing microparticles comprising a drug used to treat a skin disorder;
(b) packaging the microparticles in a container;
(c) sealing the container with a metered dose valve; and
(d) adding propellant to the container.

9. The method of claim 8 wherein the drug has PDGF-like activity.

10. The method of claim 9 wherein the drug is human recombinant PDGF-BB.

11. The method of claim 10 wherein the microparticles comprise human recombinant PDGF-BB and hydrolyzed gelatin with an average molecular weight of 20,000 daltons.

12. The method of claim 11 further comprising adding a propellant and a lubricant to the container.

13. The method of claim 12 wherein the propellant is isobutane and the lubricant is isopropyl myristate.
